# EUROPEAN PATENT APPLICATION

(11) **EP 4 687 145 A1**
(43) Date of publication of application: **04.02.2026**
(21) Application number: 24306283.3
(22) Date of filing: 30.07.2024
(51) Int. Cl.: G16C 20/10, G16C 20/70

(54) **METHOD FOR AUTOMATICALLY GENERATING AN OPTIMIZED REACTION PLAN AND CONDUCTING A MECHANOCHEMICAL REACTION**

(71) Applicant: Université de Montpellier, 34090 Montpellier (FR); Centre National de la Recherche Scientifique, 75016 Paris (FR); Ecole Nationale Supérieure de Chimie de Montpellier, 34296 Montpellier (FR)
(72) Inventor: BORDIER, Corentin, 72120 SAINT CALAIS (FR); COLACINO, Evelina, 34090 MONTPELIIER (FR)
(74) Representative: Santarelli

(57) **Abstract**

A method for automatically generating an optimized reaction plan and conducting a mechanochemical reaction in the solid state or in liquid-assisted grinding using mechanochemistry equipment such as an extruder. The method includes defining chemical and mechanical constraints and initial conditions, defining a cost function, and performing a multifactorial predictive analysis using a Bayesian optimization algorithm without prior data to optimize the cost function. The method further involves considering mechanical and geometrical parameters directly related to the equipment's mechanical components and integrating a process analytical technology (PAT) system to measure reaction data, providing in-line, real-time control and feedback. This enables the equipment to automatically and autonomously adjust parameters based on the measured reaction data.

## Description

### TECHNICAL FIELD

The present invention relates to the optimization of mechanochemical reactions in the solid state or liquid-assisted grinding using advanced computational techniques. Especially, the present invention relates to a method for automatically generating an optimized reaction plan and conducting a mechanochemical reaction in mechanochemistry equipment, such as extruders, by integrating Bayesian optimization algorithms and real-time process analytical technology (PAT) systems.

The invention has a direct, but not exclusive, application in the synthesis of active pharmaceutical ingredients (APIs). It is also applicable in other areas of chemical synthesis, where optimizing reaction conditions and improving efficiency are crucial, such as in the production of fine chemicals, materials science, and environmental chemistry.

### PRIOR ART

The field of mechanochemistry has witnessed significant advancements, particularly in the context of optimizing chemical reactions through mechanical means. Historically, mechanochemical processes have been used to induce chemical reactions by applying mechanical force, typically in solid-state environments. These processes often utilize equipment such as ball mills, grinders, and extruders, where mechanical energy is directly applied to induce reactions, thereby offering a solvent-free alternative to traditional wet chemistry methods. The evolution of mechanochemistry has been driven by the need for more efficient, cost-effective, and environmentally friendly chemical synthesis methods.

In recent years, the integration of advanced computational techniques, such as machine learning and optimization algorithms, has shown significant potential to revolutionize the field. Bayesian optimization, in particular, has emerged as a powerful tool for optimizing complex processes by iteratively adjusting process parameters to achieve desired outcomes with minimal experimentation. This technique, combined with real-time data analysis and feedback systems, has shown great potential in enhancing the efficiency and precision of mechanochemical reactions.

Several prior art documents highlight the development and application of these advanced techniques.

For instance, CN11611 0505A describes a method of optimizing continuous flow chemical processes using multi-objective Bayesian optimization to improve efficiency, safety, and automation. However, this method focuses on continuous flow chemistry and does not address the specific challenges associated with solid-state mechanochemistry or the use of extruders.

CA3185029A1 discloses a computerized method for determining target parameters in continuous flow chemistry systems, employing machine learning models for parameter optimization. This method is tailored to liquid-phase reactions and does not encompass the unique requirements of solid-state mechanochemical processes.

WO2022132050A1 discusses a system and method for processing extrusion data during an extrusion process, utilizing machine learning models for real-time optimization. Although it mentions the potential use of Bayesian optimization, it lacks specific implementation details and does not fully integrate the mechanical and geometrical parameters of the extrusion equipment into the optimization process.

JP2024003699A and EP4113351A1 describe methods for optimizing the manufacturing conditions of resin compositions using twin-screw extruders, incorporating machine learning algorithms to adjust process parameters. While these documents provide a more relevant context by addressing mechanochemical processes in extrusion equipment, they primarily focus on resin composition optimization and do not extend their application to broader mechanochemical reactions.

In addition to patent literature, scientific publications have explored various optimization strategies in solution-based chemistry. For example, "Taylor, C. J.; Pomberger, A.; Felton, K. C.; Grainger, R.; Barecka, M.; Chamberlain, T. W.; Bourne, R. A.; Johnson, C. N.; Lapkin, A. A. A Brief Introduction to Chemical Reaction Optimization. Chem. Rev. 2023, 123 (6), 3089-3126*.* "and "Shields, B.J.; Stevens, J.; Li, J.; Parasram, M.; Damani, F.; Alvarado, J. I. M.; Janey, J. M.; Adams, R. P.; Doyle, A. G. Bayesian Reaction Optimization as a Tool for Chemical Synthesis. Nature 2021, 590 (7844), 89-96*."* review optimization techniques in chemical reactions, including Bayesian optimization, but do not specifically address the integration of these techniques in mechanochemical extrusion processes.

Despite these advancements, existing solutions have limitations. Many methods are tailored to continuous flow chemistry or specific material compositions, lacking comprehensive approaches for solid-state mechanochemistry. Additionally, the integration of real-time data analysis and feedback mechanisms into the optimization process is often incomplete or inadequately detailed. These gaps highlight the need for a more robust and versatile solution that can effectively optimize mechanochemical reactions using extruders, considering both chemical and mechanical constraints and providing real-time control and feedback to enhance process efficiency and reliability.

### SUMMARY OF THE INVENTION

The aim of the present invention is to overcome some or all of the limitations of the prior art by providing a solution that optimizes mechanochemical reactions using advanced computational techniques and real-time data integration. Major advantages include enhanced efficiency and precision through Bayesian optimization, real-time adaptability with a PAT system, comprehensive consideration of mechanical and geometrical parameters, versatility in equipment and applications, and reduced manual intervention.

To this end, the object of the present invention is a method for automatically generating an optimized reaction plan and conducting a mechanochemical reaction in the solid state or in liquid-assisted grinding, using mechanochemistry equipment such as an extruder. This method comprises:
- a step of defining chemical and mechanical constraints and initial conditions;
- a step of defining a cost function; and
- a step of performing multifactorial predictive analysis by executing a Bayesian optimization algorithm without prior data to optimize the cost function.

Advantageously, the method further comprises:
- a step of integrating, in the optimization, mechanical and geometrical parameters directly related to mechanical components of the equipment; and
- a step of integrating a process analytical technology system "PAT system", measuring reaction data and providing in-line, real-time control and feedback on said method, allowing the equipment to automatically and autonomously adjust parameters based on the measured reaction data.

This method allows for the efficient and precise optimization of mechanochemical reactions, improving reaction outcomes and reducing the need for extensive trial and error.

The use of advanced computational techniques, such as Bayesian optimization, in combination with real-time data analysis and control, represents a significant advancement in the field of mechanochemistry. This approach allows for the precise and efficient optimization of complex reactions, reducing the need for manual intervention and increasing the reliability and consistency of the results.

The ability to integrate a wide range of parameters, both chemical and mechanical, into the optimization process ensures that the method can be tailored to meet the specific requirements of different reactions and applications. This flexibility, combined with the ability to continuously monitor and adjust the reaction conditions in real-time, makes the method highly effective.

The invention also offers significant economic and environmental benefits. By optimizing the reaction conditions, the method can reduce the amount of reactants and energy required, leading to cost savings and a lower environmental impact.

According to one embodiment, the mechanochemistry equipment is a single-screw, twin-screw, or multi-screw extruder, and the chemical and mechanical constraints include an extruder flow rate, a screw speed, a barrel temperature, and a residence time.

This versatility in equipment and parameter control enhances the adaptability and applicability of the method to various mechanochemical processes.

According to one embodiment, the cost function is chosen from one or a combination of the following functions: reaction duration, amount of reactants, and activation energy of the reaction.

Customizing the cost function according to specific reaction goals enables targeted optimization, leading to more efficient and economical processes.

According to one embodiment, the Bayesian optimization algorithm uses a probabilistic model updated in real time based on data collected by the PAT system.

Real-time data updates ensure continuous optimization, adapting to changing reaction conditions for improved performance and reliability.

According to one embodiment, the mechanical and geometrical parameters taken into account include at least one parameter among the geometry of the extruder screws, the internal pressure of the extruder, the mixing characteristics of the materials, the feed rate of chemical reactants forming the starting material mixture, the position of the solids feed port or liquid inlet on the barrel, the presence of a die and die type, the screw speed, the barrel temperature or individual segment temperatures, and the screw profile.

Considering a comprehensive set of parameters allows for a highly detailed and accurate optimization process, improving the overall efficiency of the mechanochemical reactions.

According to one embodiment, the PAT system is based on spectroscopic technology to measure reaction data, with the technology chosen, in a non-limiting manner, from infrared (IR) spectroscopy, near-infrared (NIR) spectroscopy, and Raman spectroscopy.

Using advanced spectroscopic techniques for PAT enables precise and accurate monitoring of reaction progress, leading to better control and optimization.

According to one embodiment, the PAT system is based on acoustic emission (AE) detection technology to measure reaction data, allowing the detection of physical changes associated with the mechanochemical reaction so that the system adjusts the mechanical parameters of the equipment in real time.

AE detection provides a non-invasive and sensitive method for monitoring physical changes, facilitating real-time adjustments and enhancing reaction control.

The present disclosure further relates to an automated mechanochemical extrusion method using an extruder, and comprising the prior generation of an optimized reaction plan according to the method as disclosed.

The present disclosure further relates to a system comprising mechanochemistry equipment and computer control means for the equipment, for implementing the disclosed method.

According to one embodiment, the mechanochemistry equipment is a single-screw, twin-screw, or multi-screw extruder.

The present disclosure further relates to a computer program product downloadable from a communication network and/or stored on a microprocessor-readable medium and executable by a microprocessor, comprising program code instructions for executing the disclosed method.

The present disclosure further relates to a non-transitory storage medium readable by a terminal, storing a computer program comprising a set of instructions executable by a computer or processor to implement the disclosed method.

The fundamental concepts of the invention having been outlined above in their most basic form, further details and characteristics will become more apparent from the following description and the accompanying drawings, which are provided by way of non-limiting example of an embodiment of a method for automatically generating an optimized reaction plan and conducting a mechanochemical reaction, in accordance with the principles of the invention.

### BRIEF DESCRIPTION OF THE FIGURES

The figures are provided purely for illustrative purposes to better understand the invention without limiting its scope. The various elements may be represented schematically.
- Figure 1: a schematic overview of the method according to the invention;
- Figure 2: a flowchart detailing the main steps of the method.

### DETAILED DESCRIPTION OF EMBODIMENTS

It should be noted that certain technical elements well known to those skilled in the art are reiterated here to avoid any insufficiency or ambiguity in the understanding of the present invention.

In the embodiment described below, reference is made to the method for automatically generating an optimized reaction plan and conducting a mechanochemical reaction, primarily intended for the synthetic of active pharmaceutical ingredients (APIs) and other chemical synthesis processes. This non-limiting example is provided for a better understanding of the invention and does not exclude its application to other mechanochemical processes or the use of different types of mechanochemistry equipment, such as single-screw, twin-screw, or multi-screw extruders.

In the present description, unless otherwise indicated, the following definitions are adopted:
- A "model" refers to an abstract concept based on data and having parameters, characterized by an output of interest obtained from input data. A typical example of a model is a machine learning model.

All other technical and scientific terms used have the same meanings as those commonly understood in the technical field of the invention. Additionally, "define," "execute," "integrate," "adjust," and their derived forms, or more broadly "executable operation" in the context of the invention, refer to an action performed by a device or processor unless the context indicates otherwise. In this regard, the operations pertain to actions and/or processes of a data processing system, such as a cloud computing system or an autonomous electronic computing device, which manipulates and transforms data represented as physical (electronic) quantities within the memories of the computing system or other storage, transmission, or display devices. These operations may be based on applications or software.

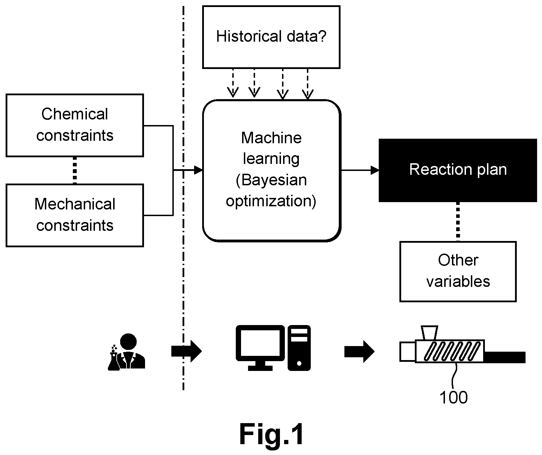

Figure 1 represents a schematic overview of the method for automatically generating an optimized reaction plan and conducting a mechanochemical reaction using an extruder or similar mechanochemistry equipment. The figure illustrates the flow of information and actions starting from the definition of chemical and mechanical constraints, through the use of machine learning, with Bayesian optimization, and culminating in the execution of the reaction plan by the extruder.

The process begins with the definition of chemical and mechanical constraints. Chemical constraints include the selection of reactants, their concentrations, and the desired products. Mechanical constraints pertain to the operational parameters of the extruder, such as screw configuration, screw speed, pressure, and temperature profiles. These constraints are crucial as they set the initial conditions for the reaction and determine the boundaries within which the optimization will operate.

The defined constraints are input into a machine learning model that employs Bayesian optimization. This optimization algorithm does not rely on prior data, allowing it to dynamically adjust and optimize the reaction plan based on real-time inputs and predictive analysis. The Bayesian model is particularly advantageous for this application because it iteratively refines its predictions and can handle complex, multidimensional optimization problems effectively.

Historical data, if available, can be fed into the Bayesian optimization model to enhance its predictive accuracy. This data may include past experiments, reaction outcomes, and operational parameters from similar reactions. By integrating historical data, the model can better predict the optimal conditions and reduce the number of iterations required to achieve the desired outcome.

The machine learning model outputs an optimized reaction plan, which includes detailed instructions for the extruder. This plan covers all relevant parameters, ensuring that the reaction is conducted under the most favorable conditions to maximize yield, efficiency, and product quality. The plan is then executed by the extruder, which is represented in Figure 1 as the final step in the process.

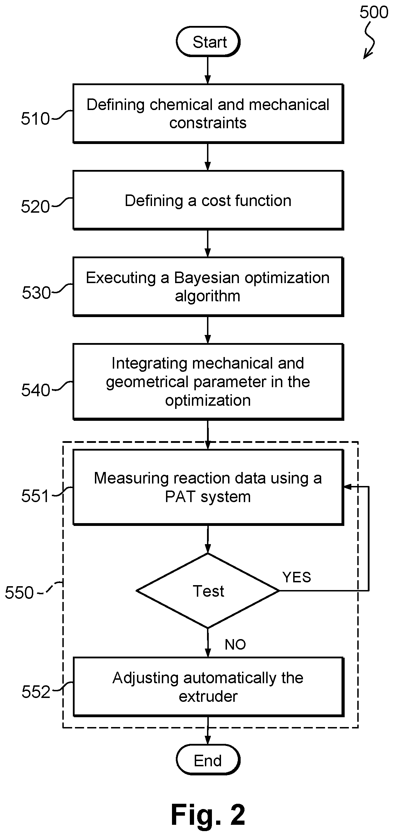

In Figure 2, the method is further detailed in a flowchart 500 that outlines each step. The method begins with step 510, where chemical and mechanical constraints are defined. This involves a thorough analysis of the reactants, the desired products, and the operational parameters of the extruder. For instance, in the synthesis of active pharmaceutical ingredients (APIs), the reactants must be chosen carefully to ensure the desired chemical reaction occurs efficiently. The mechanical constraints, such as screw speed and temperature profiles, must also be set to ensure optimal mixing and reaction conditions.

Step 520 involves defining a cost function. The cost function quantifies the objectives of the optimization, such as reaction duration, amount of reactants, and activation energy. This function guides the optimization process by providing a clear metric for evaluating different reaction conditions. For example, if minimizing reaction time is a priority, the cost function will weigh this factor more heavily.

In step 530, the Bayesian optimization algorithm is executed. This step involves running the machine learning model to predict the optimal reaction conditions. The algorithm iteratively adjusts the parameters, using real-time data and predictive analysis to refine its predictions. This approach is highly effective for optimizing complex reactions where multiple variables interact in non-linear ways.

Step 540 focuses on integrating mechanical and geometrical parameters into the optimization process. These parameters include the geometry of the extruder screws, the internal pressure, the mixing characteristics of the materials, the feed rate of chemical reactants, the position of the feed ports, the presence of a die and die type, screw speed, barrel temperature, and screw profile. By considering these parameters, the optimization process can achieve a more precise and accurate reaction plan.

Suppose the goal is to synthesize an active pharmaceutical ingredient (API) using a twin-screw extruder. The chosen reactants are a chemical precursor A and a reaction agent B. The concentration of each reactant must be carefully controlled to ensure a complete and efficient reaction while minimizing undesirable by-products.

The chemical constraints for this reaction could include:
- The purity and particle size distribution of reactants A and B.
- The concentration of the reactants to achieve the desired yield.
- The possible presence of catalysts or additives to facilitate the reaction.

The mechanical constraints could include:
- The configuration of the extruder screws, such as the length, pitch, and profile of the screws, which influence the mixing and distribution of the reactants.
- The screw speed, which mainly impacts the mechanical energy input.
- The internal pressure in the extruder, which must be controlled to avoid overpressure or underpressure conditions.
- The temperature profiles along the extruder, which need to be optimized to favor ideal reaction conditions while preventing thermal decomposition of the reactants or products.

Suppose that during the optimization process, it is discovered that increasing the concentration of reactant B improves the reaction yield. However, this increase also raises the internal temperature due to the higher exothermicity of the reaction. To compensate, it becomes necessary to adjust the extruder's temperature profile to prevent the thermal decomposition of the final product. Simultaneously, it may be necessary to modify the screw speed to increase the residence time, thereby allowing better conversion of the reactants into products.

This example illustrates how chemical and mechanical constraints are interconnected and must be dynamically balanced. Each adjustment of one constraint can have cascading effects on other parameters, necessitating a sophisticated optimization approach to navigate this complex solution space. The use of Bayesian optimization techniques and real-time process analytical technology (PAT) systems helps manage this complexity by continuously adjusting parameters to achieve optimal reaction conditions.

The PAT system is introduced in step 550. The PAT system measures reaction data in real-time, providing continuous feedback on the reaction progress. The PAT system can be based on various technologies, such as infrared (IR) spectroscopy, near-infrared (NIR) spectroscopy, Raman spectroscopy, or acoustic emission (AE) detection. These technologies allow for precise monitoring of the reaction, detecting changes in reactant concentrations, temperature, pressure, and other critical parameters.

The PAT system data is used to adjust the extruder parameters in real-time, as depicted in steps 551 and 552. If the measured data deviates from the expected values, the system automatically adjusts the operational parameters to bring the reaction back on track. This automated adjustment ensures that the reaction is conducted under optimal conditions at all times, maximizing efficiency and yield.

The PAT system utilizes various sensors and analytical techniques to continuously measure key parameters such as temperature, pressure, concentration of reactants and products, and other critical variables. For instance, the system might include infrared (IR) spectroscopy to monitor the concentration of reactants A and B in the extruder. IR spectroscopy can provide real-time data on the chemical composition of the reaction mixture by measuring the absorbance of infrared light at specific wavelengths corresponding to the reactants and products.

Additionally, the PAT system might employ near-infrared (NIR) spectroscopy or Raman spectroscopy to gain further insights into the reaction progress and the formation of the desired product. These techniques can offer complementary data, enhancing the accuracy and reliability of the measurements. For example, Raman spectroscopy could be used to detect specific molecular vibrations that indicate the formation of the API, providing a direct measurement of the product concentration.

The system could also include acoustic emission (AE) detection technology to monitor physical changes within the extruder, such as the onset of agglomeration or changes in particle size distribution. AE sensors can detect high-frequency sound waves generated by physical events in the extruder, providing early warning signs of potential issues that could affect the reaction outcome.

As the reaction progresses, the PAT system continuously collects and analyzes data from these sensors. This real-time data is fed into the optimization algorithm, which uses it to adjust the extruder's operational parameters dynamically. For instance, if the IR spectroscopy data indicates that the concentration of reactant B is dropping too quickly, the system might increase the feed rate of reactant B to maintain the optimal concentration. Similarly, if the temperature sensors detect a rise in temperature beyond the desired range, the system can adjust the cooling rate or modify the temperature profile along the extruder to prevent thermal decomposition of the API.

The integration of the PAT system with the extruder allows for immediate feedback and control. The optimization algorithm, often based on Bayesian optimization, uses the real-time data to refine its predictions and continuously improve the reaction conditions. This iterative process ensures that the reaction remains within the optimal parameter space, maximizing yield and efficiency while minimizing the production of unwanted by-products.

For example, if the PAT system detects that the reaction yield is lower than expected, it can adjust the screw speed to increase the residence time, allowing more complete conversion of the reactants. Alternatively, if the system identifies an imbalance in the reactant concentrations, it can adjust the feed rates or the position of the feed ports to ensure proper mixing and distribution within the extruder.

The method 500 can be enhanced by incorporating feedback loops that continuously refine the optimization process. For instance, after each batch of reactions, the system can analyze the results and update the machine learning model with the new data. This continuous learning process ensures that the model becomes more accurate over time, further improving the efficiency and yield of the reactions.

Alternative implementations of the invention can involve different configurations and technologies. For instance, while the primary example uses a twin-screw extruder, the method can be adapted for single-screw or multi-screw extruders. Similarly, the PAT system can utilize different spectroscopic techniques based on the specific requirements of the reaction. For example, if the reaction involves compounds that are highly sensitive to temperature changes, an IR spectroscopy-based PAT system may be more appropriate.

In addition to the primary application in synthesizing APIs and pharmaceuticals (including co-crystals and multicomponent forms), the method can be applied to other areas of chemical synthesis and pharmaceutical formulation, such as, and not exclusively the production of fine chemicals, materials science, and environmental chemistry.

The automated adjustments reduce the need for manual intervention, increasing consistency and reliability. Furthermore, the ability to handle complex, multidimensional optimization problems makes this method suitable for a wide range of applications.

The method 500 can be implemented in several ways to accommodate various requirements and constraints. For instance, the Bayesian optimization algorithm can be replaced with other machine learning techniques, such as genetic algorithms or neural networks, depending on the complexity and nature of the reaction being optimized. Similarly, the PAT system can be adapted to include other types of sensors and measurement devices, such as thermal cameras or ultrasonic sensors, to enhance the monitoring and control of the reaction process.

The system architecture can also be modified to support distributed computing environments, such as cloud-based systems, to enable more efficient data processing and storage. This would allow for the integration of large datasets and the use of advanced analytics to further improve the optimization process.

In terms of hardware, the extruder can be equipped with additional features, such as variable-speed drives, temperature-controlled zones, and high-precision feeders, to enhance its performance and flexibility. These enhancements would enable the extruder to handle a wider range of reactions and materials, making it more adaptable to different industrial applications.

Furthermore, the method can be extended to include additional steps, such as pretreatment of reactants, post-treatment of products, and in-line purification, to provide a more comprehensive and integrated solution for mechanochemical synthesis. These additional steps can be optimized using the same principles of Bayesian optimization and real-time data analysis, ensuring that the entire process is efficient.

The ability to automate the optimization and control processes also reduces the need for manual intervention, further increasing efficiency and reducing the potential for errors.

In practical applications, the method can be implemented in various industrial and research settings to optimize a wide range of mechanochemical reactions. For example, in the pharmaceutical industry, the method can be used to optimize the synthesis of active pharmaceutical ingredients (APIs) and pharmaceuticals (co-crystals, multicomponent forms), ensuring that the reactions are conducted under the most favorable conditions to maximize yield and quality. Similarly, in the production of fine chemicals, the method can be used to optimize reactions to achieve higher efficiency and yield.

In materials science, the method can be used to optimize the synthesis of new materials, ensuring that the reactions are conducted under optimal conditions to achieve the desired properties. Mechanochemistry, being an "a priori" approach, focuses on benign-by-design chemistry to prevent pollution and optimize reactions for higher efficiency while reducing the environmental impact of chemical processes. For example, in environmental applications, mechanochemistry can be used in polymer deconstruction to recover monomers, thereby contributing to waste reduction and resource recovery.

In terms of practical implementation, the system can be designed to be user-friendly, with an intuitive interface that allows operators to easily input the required constraints and parameters. The system can also provide real-time visualizations of the reaction progress, enabling operators to monitor and adjust the process as needed.

## Claims

1. A method (500) for automatically generating an optimized reaction plan and conducting a mechanochemical reaction in the solid state or in liquid-assisted grinding, said reaction using a mechanochemistry equipment (100) such as an extruder, said method comprising:
- a step (510) of defining chemical and mechanical constraints and initial conditions;
- a step (520) of defining a cost function;
- a step (530) of multifactorial predictive analysis, executing a Bayesian optimization algorithm without prior data to optimize the cost function;
and **characterized in that** it further comprises:
- a step (540) of integrating, in the optimization, mechanical and geometrical parameters directly related to mechanical components of the equipment; and
- a step (550) of integrating a process analytical technology system "PAT system", measuring reaction data and providing in-line, real-time control and feedback on said method, allowing the equipment (100) to automatically and autonomously adjust parameters based on the measured reaction data.

2. The method according to claim 1, wherein the mechanochemistry equipment (100) is a single-screw, twin-screw, or multi-screw extruder, and wherein the chemical and mechanical constraints comprise an extruder flow rate, a screw speed, a barrel temperature and a residence time.

3. The method according to claim 1 or 2, wherein the cost function is chosen from one or a combination of the following functions: reaction duration, amount of reactants, and activation energy of the reaction.

4. The method according to any one of the preceding claims, wherein the Bayesian optimization algorithm uses a probabilistic model updated in real time based on data collected by the PAT system.

5. The method according to any one of the preceding claims, wherein the mechanical and geometrical parameters taken into account comprise the geometry of the extruder screws, the internal pressure of the extruder, the mixing characteristics of the materials, the feed rate of chemical reactants forming the starting material mixture, the position of the solids feed port or liquid inlet on the barrel, the presence of a die and die type, the screw speed, the barrel temperature or individual segment temperatures, and the screw profile.

6. The method according to any one of the preceding claims, wherein the PAT system is based on spectroscopic technology to measure reaction data, said technology being chosen from infrared (IR) spectroscopy, near-infrared (NIR) spectroscopy, and Raman spectroscopy.

7. The method according to any one of the preceding claims, wherein the PAT system is based on acoustic emission (AE) detection technology to measure reaction data, said technology allowing the detection of physical changes associated with the mechanochemical reaction so that said system adjusts the mechanical parameters of the equipment (100) in real time.

8. An automated mechanochemical extrusion method, implementing an extruder (100), said method comprising the prior generation of an optimized reaction plan according to the method (500) of any one of the preceding claims.

9. A system (200) comprising a mechanochemistry equipment (100) and computer control means for said equipment, for implementing a method (500) according to any one of claims 1 to 7.

10. The system according to claim 9, wherein the mechanochemistry equipment (100) is a single-screw, twin-screw, or multi-screw extruder.

11. A computer program product downloadable from a communication network and/or stored on a microprocessor-readable medium and executable by a microprocessor, **characterized in that** it comprises program code instructions for executing a method (500) according to any one of claims 1 to 7.

12. A non-transitory storage medium readable by a terminal, storing a computer program comprising a set of instructions executable by a computer or processor to implement a method (500) according to any one of claims 1 to 7.
